(19) Europäisches Patentamt
European Patent Office
Office européen des brevets

(11) Veröffentlichungsnummer: **0 034 206**
**B1**

(12) # EUROPÄISCHE PATENTSCHRIFT

(45) Veröffentlichungstag der Patentschrift: **13.04.83**

(51) Int. Cl.³: **C 07 D 303/04,** C 07 D 303/06, C 07 D 301/16

(21) Anmeldenummer: **80106976.6**

(22) Anmeldetag: **12.11.80**

(54) **Verfahren zur Epoxydierung von Cyclododecen oder Tricyclodecen-3.**

(30) Priorität: **26.01.80 DE 3002811**

(43) Veröffentlichungstag der Anmeldung: **26.08.81 Patentblatt 81/34**

(45) Bekanntmachung des Hinweises auf die Patenterteilung: **13.04.83 Patentblatt 83/15**

(84) Benannte Vertragsstaaten: **AT BE CH FR GB IT LI NL SE**

(56) Entgegenhaltungen:
**AT-B-187 900**
**DE-C-962 073**
**US-A-3 130 207**
**Methodicum Chimicum (1975) Georg Thieme Verlag Stuttgart, S. 170**
**Chemical Abstracts, Band 50, Nr. 15, 10. August 1956 Columbus, Ohio, USA V. PRELOG et al. »Carbon rings. LXIX. The reaction of cyclododecenes with peroxyformic acid« Spalte 10 665e**
**Chemical Abstracts, Band 41, Nr. 21, 10. November 1947 Columbus, Ohio, USA D. SWERN »*Electronic interpretation of the reaction of* olefins with organic per acids« Spalte 6870f.**

(73) Patentinhaber: **Degussa Aktiengesellschaft, Weissfrauenstrasse 9, D-6000 Frankfurt am Main 1 (DE)**

(72) Erfinder: **Käbisch, Gerhard, Dr., Palmstrasse 1, D-7888 Rheinfelden 5 (DE)**
Erfinder: **Trübe, Rudolf, Ernst-Reuter-Strasse 22, D-7888 Rheinfelden (DE)**
Erfinder: **Wittmann, Hans, Liebenbergstrasse 12, D-7888 Rheinfelden 8 (DE)**
Erfinder: **Raupach, Siegfried, Langentalstrasse 24, D-7888 Rheinfelden (DE)**
Erfinder: **Malitius, Horst, Blümleacker 5, D-7888 Rheinfelden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Verfahren zur Epoxydierung von Cyclododecen oder Tricyclodecen-3

Die Erfindung betrifft ein Verfahren zur Epoxydierung von Cyclododecen oder Tricyclodecen-3.

Nach einem Übersichtsartikel in Chemical Week (1963, 6. April, Seiten 55 bis 64) lassen sich mit in situ aus Ameisensäure und Wasserstoffperoxid gebildeter Perameisensäure nur leicht reagierende Verbindungen, wie natürliche Fette und Öle oder lineare Polybutadiene, epoxydieren, wohingegen zur Epoxydierung anderer Olefine höhere, meist vorgefertigte Persäuren erforderlich sind.

Dementsprechend ist beispielsweise auch bereits ein Verfahren zur Epoxydierung von Tricyclodecen-3 bekannt, bei dem das Olefin in Eisessiglösung mit vorgefertigter Peressigsäure umgesetzt wird (AT-AF-187 900). Dieses aufwendige Verfahren hat jedoch, ganz offensichtlich aus wirtschaftlichen Gründen, niemals Eingang in die Praxis gefunden.

Andererseits ist in Helv. Chim. Acta 38, Seiten 1786 bis 1794 (1955) die Umsetzung von Cyclododecen mit in situ aus Ameisensäure und Wasserstoffperoxid gebildeter Perameisensäure beschrieben. Unter den angewandten Reaktionsbedingungen werden aber als Endprodukte die vicinalen Cyclododecandiole erhalten.

In Methodicum Chimicum (1975), Georg Thieme Verlag Stuttgart, Seite 170, wird generell vor der Anwendung erhöhter Reaktionstemperaturen bei der Epoxydierung von Olefinen mittels Persäuren gewarnt. In der dort indirekt zitierten DE-C-962 073 wird dann auch bestätigt, daß bei der Epoxydierung von Cycloocten mittels in situ gebildeter Perameisensäure die Erhöhung der Reaktionstemperatur zu einer Verminderung der Ausbeute an Epoxid führt.

Das erfindungsgemäße Verfahren ist nun dadurch gekennzeichnet, daß man das Olefin zusammen mit Ameisensäure einer Konzentration von mindestens 70 Gewichtsprozent in einer Menge von weniger als 1 Mol pro Mol zu epoxydierender Doppelbindung vorlegt und bei einer Temperatur zwischen 45 und 65° C Wasserstoffperoxid einer Konzentration von mindestens 20 Gewichtsprozent in einer Menge von mindestens 1,05 Mol pro Mol zu epoxydierender Doppelbindung während eines Zeitraumes von mindestens 2 Stunden zudosiert.

Vorzugsweise wird eine Ameisensäure mit einer Konzentration von 85 bis 100 Gewichtsprozent eingesetzt. Die bevorzugten Einsatzmengen an Ameisensäure liegen zwischen 0,2 und 0,8 Mol zu epoxydierender Doppelbindung. Das Wasserstoffperoxid wird vorzugsweise mit einer Konzentration von 30 bis 75 Gewichtsprozent eingesetzt. Die bevorzugten Einsatzmengen an Wasserstoffperoxid liegen zwischen 1,3 und 1,55 Mol pro Mol zu epoxydierender Doppelbindung.

Unerwarteterweise lassen sich die Olefine durch das erfindungsgemäße Verfahren trotz Anwendung einer relativ hohen Reaktionstemperatur in relativ kurzer Reaktionszeit bei hohen Umsätzen in guten Ausbeuten in die entsprechenden Epoxide umwandeln.

Um die Temperatur in dem vorgeschriebenen Bereich halten zu können, muß, insbesondere bei größeren Ansätzen, für Möglichkeiten zu einer ausreichenden Abführung der freiwerdenden Reaktionswärme gesorgt werden. Zweckmäßigerweise wird das erfindungsgemäße Verfahren in Abwesenheit von zusätzlichen Lösungsmitteln und von Katalysatoren für die Bildung der Perameisensäure aus der Ameisensäure und dem Wasserstoffperoxid durchgeführt.

Das Wasserstoffperoxid wird während eines Zeitraumes zwischen mindestens 2 Stunden und höchstens 8 Stunden zudosiert. Einschließlich einer angemessenen Nachreaktionszeit kann dann die für die Epoxydierung insgesamt erforderliche Reaktionszeit auf etwa 10 Stunden oder weniger beschränkt werden. Daraus ergibt sich eine durchaus annehmbare Raum-Zeit-Ausbeute. Bei ausreichend dimensionierten Kühlflächen ist die Gesamtreaktionszeit (=Zudosierungs- und Nachreaktionszeit) umso kürzer, je höher die Konzentrationen des eingesetzten Wasserstoffperoxids und der eingesetzten Ameisensäure sind. Die Ameisensäure kann in der ganzen vorgesehenen Menge zusammen mit dem Olefin vorgelegt werden. Ebensogut ist es aber auch möglich, zunächst nur einen Teil der Ameisensäure vorzulegen und den Rest während der Umsetzung hinzuzufügen.

Das erfindungsgemäße Verfahren ist auch in großtechnischen Produktionsanlagen gefahrlos durchzuführen, wenn einige sicherheitstechnische Maßnahmen ergriffen werden. So können z. B. automatische Sicherheitsventile vorgesehen werden, die sich öffnen und das Reaktionsgemisch mit Wasser verdünnen, wenn die Mischorgane ausfallen, die Menge des durch Zersetzung von Wasserstoffperoxid oder Perameisensäure gebildeten Abgases einen vorgegebenen Grenzwert überschreitet oder die Temperatur merklich über 65° C anzusteigen droht.

Nach beendeter Epoxydierungsreaktion wird im Normalfall die wäßrige Phase von der organischen Epoxidphase getrennt. Zur weiteren Reinigung empfiehlt es sich, die organische Phase von darin noch gelösten Wasserstoffperoxid- und Ameisensäure-Anteilen zu befreien. Bevorzugt wird hierzu ein zwei- bis dreimal hintereinander vorgenommenes Auswaschen mit Wasser, wobei das Volumenverhältnis zwischen der gegebenenfalls mit einem inerten Lösungsmittel, wie Ethylacetat, verdünnten, organischen Phase und dem Waschwasser jeweils etwa 10 : 1 bis 2 : 1 beträgt. Wegen der geringen Wasserlöslichkeit der gebildeten Epoxide kann das Auswaschen auch bei erhöhten Temperaturen vorgenommen werden. Die organische Phase muß daher nach beendeter Epoxydierungsreaktion nicht erst abgekühlt werden. Das nach dem letzten Auswaschen in der organischen Phase gegebenenfalls enthaltene Lösungsmittel und/oder das darin gelöste Wasser kann leicht, vorzugsweise unter

vermindertem Druck, destillativ entfernt werden.

Die nach dem erfindungsgemäßen Verfahren erhältlichen Epoxide, nämlich 1,2-Epoxycyclododecan und 3,4-Epoxytricyclodecan sind wertvolle Zwischenprodukte für Synthesen. Sie sind im allgemeinen so rein, daß sie für die meisten Zwecke direkt eingesetzt werden können. Sollte dies in speziellen Fällen erwünscht sein, können sie aber natürlich auch in an sich bekannter Weise, z. B. durch fraktionierte Destillation unter vermindertem Druck, weiter gereinigt werden.

Die nach der Abtrennung der organischen Phase hinterbleibende abgereicherte wäßrige Phase kann in an sich bekannter Weise zur Rückgewinnung des enthaltenen Wasserstoffperoxids aufgearbeitet werden.

Das erfindungsgemäße Verfahren soll durch die nachfolgenden Beispiele näher erläutert werden:

## Beispiel 1

In einem 1-l-Dreihalskolben mit Rührer, Rückflußkühler, Tropftrichter und Thermometer werden vorgelegt

| | |
|---|---|
| 166,4 g (1,0 Mol) | Cyclododecen und |
| 33,0 g (0,7 Mol) | Ameisensäure (rein). |

Unter intensiver Rührung und Kühlung werden im Verlaufe von 4 Stunden

| | |
|---|---|
| 256,0 g (1,5 Mol) | Wasserstoffperoxid (20 Gewichtsprozent) |

zudosiert.

Während der Zudosierung des Wasserstoffperoxids und einer anschließenden Nachreaktionszeit von weiteren 4 Stunden wird die Temperatur im Inneren des Kolbens auf etwa 55° C gehalten.

Nach insgesamt 8 Stunden wird die Umsetzung abgebrochen und die wäßrige Phase abgetrennt. Sie enthält noch 8,3 Gewichtsprozent Ameisensäure und 7,3 Gewichtsprozent Wasserstoffperoxid.

Die verbleibende organische Phase (172 g) wird dreimal hintereinander mit je 100 ml Wasser gewaschen. Die danach durchgeführte Analyse ergibt folgende Zusammensetzung:

71,2 Gewichtsprozent 1,2-Epoxycyclododecan
22,5 Gewichtsprozent nicht umgesetztes Cyclododecen
6,3 Gewichtsprozent nicht identifizierte, hochsiedende Nebenprodukte.

Bei der destillativen Aufarbeitung unter vermindertem Druck (12 Torr) geht das 1,2 Epoxycyclododecan bei ca. 130° C über.

## Beispiel 2

In einem 1-l-Dreihalskolben mit Rührer, Rückflußkühler, Tropftrichter und Thermometer werden vorgelegt

| | |
|---|---|
| 166,4 g (1,0 Mol) | Cyclododecen und |
| 16,5 g (0,35 Mol) | Ameisensäure (98 Gewichtsprozent). |

Unter intensiver Rührung und Kühlung werden im Verlaufe von 3 Stunden

| | |
|---|---|
| 115,0 g (1,3 Mol) | Wasserstoffperoxid (40 Gewichtsprozent) |

zudosiert.

Während der Zudosierung des Wasserstoffperoxids und einer anschließenden Nachreaktionszeit von weiteren 4 Stunden wird die Temperatur im Inneren des Kolbens auf etwa 55° C gehalten. Nach dem Abbruch der Reaktion wird in der wäßrigen Phase ein Wasserstoffperoxid-Restgehalt von 11,5 Gewichtsprozent gemessen.

Die organische Phase wird wie im Beispiel 2 ausgewaschen und unter vermindertem Druck (12 Torr) destilliert. Dabei werden erhalten:

| | |
|---|---|
| Fraktion 1 (bis 125° C): | 33 g Cyclododecen |
| Fraktion 2 (125 bis 135° C): | 130 g 1,2-Epoxycyclododecan |
| Rückstand: | 9 g (nicht identifiziert). |

## Beispiel 3

In einem 1-l-Dreihalskolben mit Rührer, Rückflußkühler, Tropftrichter und Thermometer werden vorgelegt

268,0 g (2,0 Mol) Tricyclodecen-3 der Formel

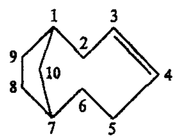

84,6 g Ameisensäure (98 Gewichtsprozent) und
0,3 g des Natriumsalzes der Hydroxyethandiphosphonsäure.

Unter intensiver Rührung und Außenkühlung zur Abführung der freiwerdenden Reaktionswärme werden im Verlaufe von 5 Stunden

510,0 g (3,0 Mol) Wasserstoffperoxid (20 Gewichtsprozent)

zudosiert.

Während der Zudosierung des Wasserstoffperoxids und einer anschließenden Nachreaktionszeit von weiteren 2 Stunden wird die Temperatur im Inneren des Kolbens bei 55°C gehalten. Nach beendeter Umsetzung wird aus dem zweiphasigen Gemisch noch heiß die wäßrige Phase abgezogen. Da die organische Phase bei etwa 55°C bereits nach kurzer Zeit eine pastenartige Form annimmt, wird sie für die anschließenden Wasserwäschen (dreimal hintereinander mit je 50 ml Wasser) mit 500 ml Ethylacetat verdünnt.

Bei der destillativen Aufarbeitung der organischen Phase wird zunächst das Ethylacetat zurückgewonnen. Bei vermindertem Druck (12 Torr) werden anschließend folgende Fraktionen erhalten:

| | |
|---|---|
| Fraktion 1 (bis 100°C): | 9 g (nicht umgesetztes Olefin) |
| Fraktion 2 (100 bis 108°C): | 280 g 3,4-Epoxytricyclodecan |
| Rückstand: | 12 g (Polymere). |

Die Fraktion 2 weist einen Erstarrungspunkt von ca. 88°C auf und hat einen Epoxidgehalt von etwa 92% der Theorie.

## Beispiel 4

In einem 1-l-Dreihalskolben mit Rührer, Rückflußkühler, Tropftrichter und Thermometer werden vorgelegt

166,4 g (1,0 Mol) Cyclododecen und
9,2 g (0,2 Mol) Ameisensäure (98 Gewichtsprozent).

Unter intensiver Rührung und Kühlung werden im Verlaufe von 4 Stunden

48,0 g (1,2 Mol) Wasserstoffperoxid (85 Gewichtsprozent)

zudosiert.

Während der Zudosierung des Wasserstoffperoxids und einer anschließenden Nachreaktionszeit von einer weiteren Stunde wird die Temperatur im Innern des Kolbens bei 50°C gehalten. Nach dem Abbruch der Reaktion und Aufarbeitung der organischen Phase wie im Beispiel 2 wird folgende Zusammensetzung durch Gaschromatographie ermittelt:

76 Gewichtsprozent 1,2-Epoxycyclododecan
24 Gewichtsprozent nicht umgesetztes Cyclododecen.

Das Gemisch kann wie im Beispiel 2 durch fraktionierte Destillation getrennt werden.

**Patentansprüche**

1. Verfahren zur Epoxydierung von Cyclododecen oder Tricyclodecen-3, dadurch gekennzeichnet, daß man das Olefin zusammen mit Ameisensäure einer Konzentration von mindestens 70 Gewichtsprozent in einer Menge von weniger als 1 Mol pro Mol zu epoxydierender Doppelbindung vorlegt und bei einer Temperatur zwischen 45 und 65°C Wasserstoffperoxid einer Konzentration von mindestens 20 Gewichtsprozent in einer Menge von mindestens 1,05 Mol pro Mol zu epoxydierender Doppelbindung während eines Zeitraumes von mindestens 2 Stunden zudosiert.
2. Verfahren nach Anspruch 1, dadurch gekennzeichnet, daß man eine Ameisensäure mit einer Konzentration von 85 bis 100 Gewichtsprozent einsetzt.
3. Verfahren nach Anspruch 1 oder 2, dadurch gekennzeichnet, daß man die Ameisensäure in einer Menge zwischen 0,2 und 0,8 Mol pro Mol zu epoxydierender Doppelbindung einsetzt.
4. Verfahren nach einem der Ansprüche 1 bis 3, dadurch gekennzeichnet, daß man Wasserstoffperoxid mit einer Konzentration von 30 bis 75 Gewichtsprozent einsetzt.
5. Verfahren nach einem der Ansprüche 1 bis 4, dadurch gekennzeichnet, daß man das Wasserstoffperoxid in einer Menge zwischen 1,3 und 1,55 Mol pro Mol zu epoxydierender Doppelbindung einsetzt.

**Claims**

1. A process for epoxidising cyclododecene or tricyclodecene-3, characterised in that the olefin is introduced together with formic acid of a concentration of at least 70% by weight in a quantity of less than 1 mol per mol of double bond to be epoxidised, and hydrogen peroxide of a concentration of at least 20% by weight is metered in at a temperature of from 45 to 65°C in a quantity of at least 1.05 mols per mol of double bond to be epoxidised over a period of at least 2 hours.
2. A process according to claim 1, characterised in that a formic acid of a concentration of from 85 to 100% by weight is used.
3. A process according to claim 1 or 2, characterised in that the formic acid is used in a quantity of from 0.2 to 0.8 mols per mol of double bond to be epoxidised.
4. A process according to one of claims 1 to 3, characterised in that hydrogen peroxide of a concentration of from 30 to 75% by weight is used.
5. A process according to one of claims 1 to 4, characterised in that the hydrogen peroxide is used in a quantity of from 1.3 to 1.55 mols per mol of double bond to be epoxidised.

**Revendications**

1. Procédé d'époxydation de cyclododécéne ou de tricyclodécéne-3, caractérisé en ce que l'on introduit l'oléfine, en commun avec de l'acide formique d'une concentration d'au moins 70% en poids, en proportion inférieure à 1 mole par mole de liaison double à époxyder, et l'on y introduit, en l'espace d'au moins 2 heures, à une température comprise entre 45 et 65°C; du peroxyde d'hydrogène, sous forme d'eau oxygénée à au moins 20% en poids, à raison d'au moins 1,05 mole par mole de liaison double à époxyder.
2. Procédé selon la revendication 1, caractérisé en ce que l'on utilise de l'acide formique dont la concentration est de 85 à 100% en poids.
3. Procédé selon l'une des revendications 1 ou 2, caractérisé en ce que l'on met en oeuvre l'acide formique en une proportion comprise entre 0,2 et 0,8 mole par mole de double liaison à époxyder.
4. Procédé selon l'une quelconque des revendications 1 à 3, caractérisé en ce que l'on met en oeuvre du peroxyde d'hydrogène sous forme d'eau oxygénée à la concentration de 30 à 75% en poids.
5. Procédé selon l'une quelconque des revendications 1 à 4, caractérisé en ce que l'on met en oeuvre le peroxyde d'hydrogène en une proportion comprise entre 1,3 et 1,55 mole par mole de double liaison à époxyder.